# EUROPÄISCHE PATENTSCHRIFT

(11) **EP 0 613 923 B1**
(45) Veröffentlichungstag und Bekanntmachung des Hinweises auf die Patenterteilung: **24.10.2001**
(21) Anmeldenummer: 94102683.3
(22) Anmeldetag: 23.02.1994
(51) Int. Cl.: C08K 5/15, C08L 23/06, C08J 3/22, A61F 2/00

(54) **Stabilisierte Formmasse aus Polyethylen, ein Verfahren zur Herstellung und Verwendung**
Stabilized polyethylen moulding composition, process of preparation and its use
Composition de moulage stabilisée en polyéthylène, procédé de préparation et son utilisation

(30) Priorität: 03.03.1993 DE 4306593
(43) Veröffentlichungstag der Anmeldung: 07.09.1994
(73) Patentinhaber: Ticona GmbH, 65451 Kelsterbach (DE)
(72) Erfinder: Berzen, Josef, Dipl.-Phys., D-46147 Oberhausen (DE); Luketic, Dragan, D-46147 Oberhausen (DE)

(56) Entgegenhaltungen:
- EP-A- 0 263 524
- EP-A- 0 542 108
- JP-A- 54 034 352
- EUROPEAN PLASTICS NEWS Bd. 20, Nr. 8 , 1993 , CROYDON GB Seite 26 'Antioxidant mimics nature' & KONFERENZ "COMPOUNDING '93' 4. Februar 1993 , BRüSSEL BE

## Beschreibung

Die Erfindung betrifft eine stabilisierte Formmasse aus Polyethylen mit einer mittleren Molmasse von 10⁵ bis 10⁷ g/mol, ein Verfahren zur Herstellung und Verwendung.

Formmassen aus Polyethylen, gegebenenfalls verstärkt mit anorganischen Fasern oder Pulvern gehören zum Stand der Technik. Diese Formmassen haben zahlreiche günstige physikalische und chemische Eigenschaften, wie beispielsweise niedrige Reibungskoeffizienten, hoher Verschleißwiderstand bei hoher Elastizität oder Chemiekalienbeständigkeit.

Mit steigendem Polymerisationsgrad werden darüber hinaus das Zähigkeits- und Verschleißverhalten sowie seine bemerkenswerte Beständigkeit gegenüber zahlreichen Chemikalien im Vergleich zu anderen Werkstoffen deutlich verbessert.

Nachteilig beim Polyethylen ist seine Empfindlichkeit gegen Wärme-, Licht- und Oxidationseinflüsse. Diese Empfindlichkeit kann durch Zugabe von Additiven in Mengen von 0,1 bis 0,2 Gew%, bezogen auf das Polyethylen, teilweise beseitigt werden. Als Additive haben sich beispielsweise 4,4'-Thio-bis-(3-methyl-6-tertiärbutylphenol-1), Dilauryl-thiodipropionat, Distearyl-thio-dipropionat, Tetrakis-[methylen-(3,5-ditertiärbutyl-4-hydroxy-hydrocinnamat)]-methan, n-Octadecyl-β-(4'-hydroxy-3,5'-ditertiärbutyl-phenyl)-propionat, Bis-[3,3-bis(hydroxy-3'-tertiärbutyl-phenyl)-butansäure]-glykolester bewährt.

Bei langer Nutzungsdauer von Polyethylen ist insbesondere die Oxidationsstabilität nicht ausreichend. Die mangelnde Oxidationsstabilität führt zu einem Kettenabbau mit der Folge, daß sich sein Verschleiß erhöht und die mechanischen Eigenschaften, wie z.B. vermindertes Festigkeitsverhalten, merklich beeinträchtigt werden. Bei Anwendungen mit hoher Standzeitforderung, wie z.B. bei der Verwendung als Implantatwerkstoff für künstliche Gelenke der Hüfte, Schulter, des Knies usw. aus vornehmlich ultrahochmolekularem Polyethylen (PE-UHMW) mit hohem Reinheitsgrad kommt es bedingt durch die aggressive Körperflüssigkeit und den im Gewebe befindlichen Sauerstoff aufgrund von Oxidationsprozessen im Laufe der Jahre zu einem molekularen Abbau des PE-UHMW, was den Verschleiß erhöht und demzufolge einen Austausch des Implantats nötig macht.

Es war daher die Aufgabe gestellt, die Oxidationsbeständigkeit des Polyethylens zu erhöhen.

Überraschend konnte diese Aufgabe durch die gemeinsame Einarbeitung bestimmter Antioxidantien und α -Tocopherol oder dessen Abkömmling gelöst werden. Bei der gemeinsamen Einarbeitung bestimmter Antioxidantien und Tocopherol, insbesondere α-Tocopherol oder Vitamin E als Abkömmling vom α-Tocopherol kommt es zu einem Synergismus, der zu einer starken Erhöhung der Oxidationsbeständigkeit von Polyethylen führt.

Die erfindungsgemäße stabilisierte Formmasse aus Polyethylen mit einer mittleren Molmasse von 10⁵ bis 10⁷ g/mol enthält somit
a) 0,01 bis 1 Gew% eines Zinksalzes vom Dialkyl-di-thiocarbamat mit C₆ bis C₁₆-Ketten in der Alkylgruppe als Antioxidans und 0,01 bis 0,5 Gew% α-Tocopherol oder dessen Abkömmling als Stabilisator;
b) 3 bis 40 Gew% Fasern und/oder Pulver aus Graphit, Glas, Quarz oder Metalle als Füllstoff und gegebenenfalls weitere übliche Additive, wie beispielsweise Farboigmente, Gleitmittel, Verarbeitungsstabilisatoren oder Flammschutzmittel.

Die Herstellung der erfindungsgemäßen stabilisierten Formmasse erfolgt dadurch, daß man
a) alle Inhaltsstoffe gemeinsam mischt und die Mischung unter Drucken von etwa 3 bis 5 MPa bei Temperaturen von etwa 180 bis 250°C sintert und das Sinterprodukt unter Drucken von etwa 7 bis 10 MPa abkühlt (Sinter- und Kühlzeit hängen weitgehend von der Materialdicke ab); oder
b) zunächst für ein Masterbatch aus 0,1 bis 10 Gew% Antioxidantien, 0,1 bis 5 Gew% Stabilisator und Rest Polyethylen die Einzelkomponenten vermischt und mit weiterem Polyethylen die entsprechende Konzentration einstellt und gegebenenfalls mit weiteren Zuschlagstoffen vermischt und diese Mischung bei Drucken von 3 bis 5 MPa bei Temperaturen von 180 bis 250°C sintert und unter Drucken von 7 bis 10 MPa abkühlt.

Die erfindungsgemäße stabilisierte Formmasse läßt sich in üblicher Art durch Pressen, Stanzen, Sägen oder Bohren der Anwendungsform anpassen.

Neben dem Einsatz als Implantat kann die stabilisierte Formmasse insbesondere im technischen Bereich z.B. für langlebige Gleit-und Wälzlager verwendet werden.

Obwohl die Erfindung auf alle Polyethylenqualitäten mit Erfolg angewendet werden kann, ist die Anwendung insbesondere in Polyethylen mit einer mittleren Molmasse von 2,5 . 10⁶ bis 10⁷ g/mol besonders geeignet. Polyethylen mit einer mittleren Molmasse von 2,5 . 10⁶ bis 10⁷ g/mol wird als "Polyethylen mit ultrahohem Molekulargewicht" gehandelt.

Zur Messung der Oxidationsstabilisierung von Polyethylen wurde folgender Test angewendet:

Die Bestimmung der Sauerstoffinduktionszeit (OIT-Wert) erfolgt mit dem DSC-Gerät TA 3000 der Fa. Mettler. In einem offenen Tiegel aus Aluminium wurden ca. 15 mg der zu prüfenden Formmasse eingewogen. Dieser Tiegel wird in die mit 4,5 l/h Stickstoff gespülte Meßzelle der Apparatur bei 35 °C eingesetzt. Die Messung wird bei 200 °C unter Sauerstoffzufuhr von 3 l/h durchgeführt, um eine schnellere oxidative Schädigung der Formmasse zu erreichen. Die Induktionszeit als Maß für die gewählte Stabilisierung der Formmasse wird aus der Auftragung des DSC-Signals gegen die Meßzeit im Wendepunkt der Meßkurve rechnerisch ermittelt.

In der Tabelle sind die Meßwerte der Oxidationsstabilität zusammengefaßt.

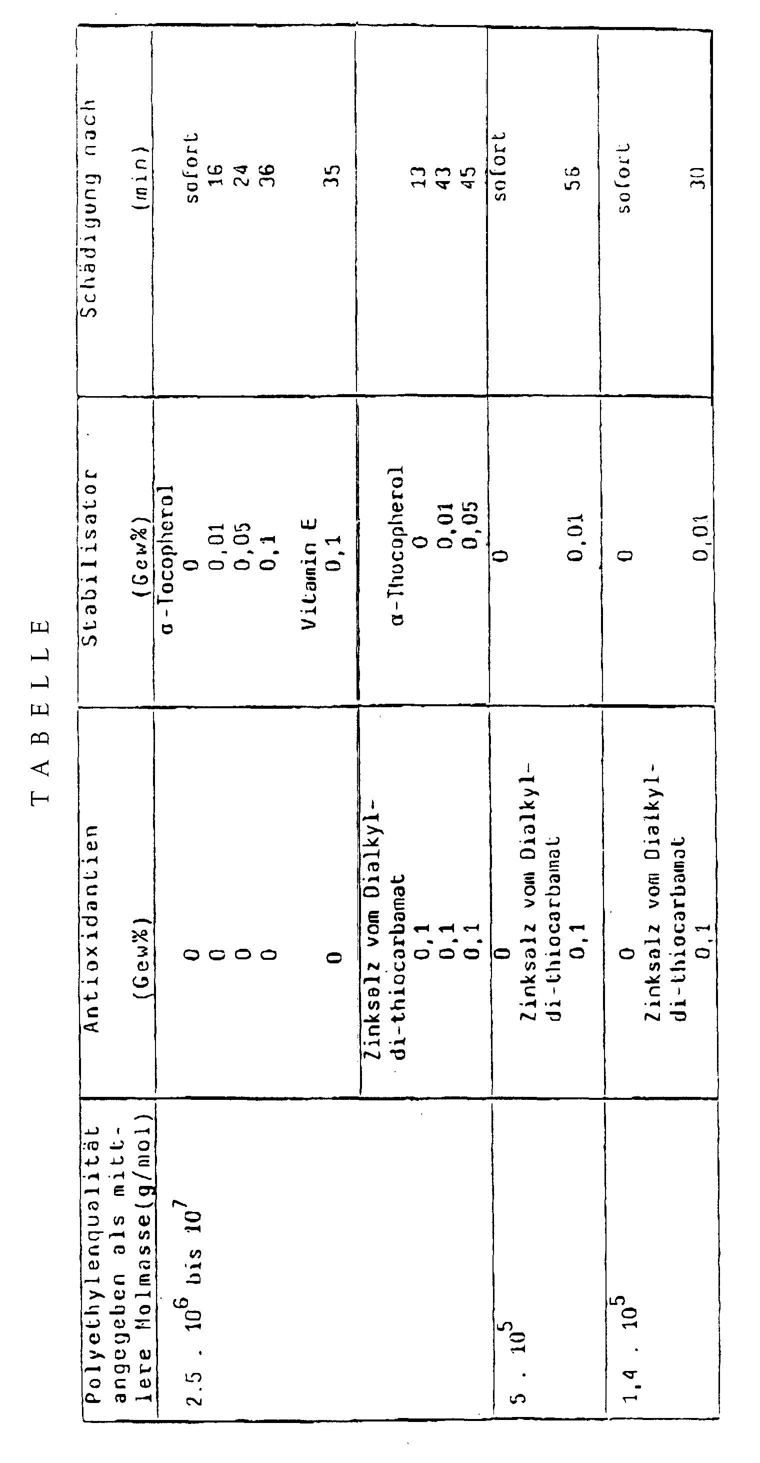

## Patentansprüche

1. Stabilisierte Formmasse aus Polyethylen mit einer mittleren Molmasse von 10⁵ bis 10⁷ g/mol enthaltend ein Zinksalz von Dialkyl-dithiocarbamat mit C₆- bis C₁₆-Ketten in der Alkylgruppe als Antioxidans und α-Tocopherol oder dessen Abkömmling als Stabilisator.

2. Stabilisierte Formmasse nach Anspruch 1, **dadurch gekennzeichnet, daß** die Formmasse 0,01 bis 1 Gew% eines Zinksalzes vom Dialkyldi-thiocarbamat mit C₆ bis C₁₆-Ketten in der Alkylgruppe als Antioxidans und 0,01 bis 0,5 Gew% α-Tocopherol oder dessen Abkömmling als Stabilisator enthält.

3. Stabilisierte Formmasse nach Anspruch 1 oder 2, **dadurch gekennzeichnet, daß** die Formmasse 3 bis 40 Gew% Fasern und/oder Pulver aus Graphit, Glas, Quarz oder Metallen als Füllstoff und gegebenenfalls weitere übliche Additive, wie Farbpigmente, Gleitmittel, Verarbeitungsstabilisatoren oder Flammschutzmittel enthält.

4. Verfahren zur Herstellung der Formmasse nach einem der Ansprüche 1 bis 3, **dadurch gekennzeichnet, daß** man alle Inhaltsstoffe gemeinsam mischt und die Mischung unter Drucken von etwa 3 bis 5 MPa bei Temperaturen von etwa 180 bis 250°C sintert und das Sinterprodukt unter Drucken von etwa 7 bis 10 MPa abkühlt.

5. Verfahren zur Herstellung der Formmasse nach einem der Ansprüche 1 bis 3, **dadurch gekennzeichnet, daß** man zunächst für ein Masterbatch aus 0,1 bis 10 Gew% Antioxidantien, 0,1 bis 5 Gew% Stabilisator und Rest Polyethylen die Einzelkomponenten vermischt und mit weiterem Polyethylen die gewünschte Konzentration einstellt und gegebenenfalls mit weiteren Zuschlagstoffen vermischt und diese Mischung bei Drucken von 3 bis 5 MPa bei Temperaturen von 180 bis 250°C sintert und unter Drucken von 7 bis 10 MPa abkühlt.

## Claims

1. A stabilized molding composition of polyethylene having an average molecular weight of 10⁵ to 10⁷ g/mol comprising a zinc salt of dialkyl dithiocarbamate having C₆ to C₁₆ chains in the alkyl group as the antioxidant and α-tocopherol or a derivative thereof as the stabilizer.

2. A stabilized molding composition as claimed in claim 1, wherein the molding composition comprises 0.01 to 1 % by weight of a zinc salt of the dialkyl dithiocarbamate having C₆ to C₁₆ chains in the alkyl group as the antioxidant and 0.01 to 0.5 % by weight of α-tocopherol or a derivative thereof as the stabilizer.

3. A stabilized molding composition as claimed in claim 1 or 2, wherein the molding composition comprises 3 to 40 % by weight of fibers and/or powder of graphite, glass, quartz or metals as the filler, and if appropriate other customary additives, such as color pigments, lubricants, processing stabilizers or flameproofing agents.

4. A process for the preparation of a molding composition as claimed in one of claims 1 to 3, which comprises mixing all the ingredients together, sintering the mixture under pressures of about 3 to 5 MPa at temperatures of about 180 to 250° C and cooling the sintered product under pressures of about 7 to 10 MPa.

5. A process for the preparation of a molding composition as claimed in one of claims 1 to 3, which comprises first mixing the individual components for a masterbatch of 0.1 to 10 % by weight of antioxidant, 0.1 to 5 % by weight of stabilizer and polyethylene as the remainder, adjusting the masterbatch to the appropriate concentration with further polyethylene and if appropriate mixing it with further additives, and sintering this mixture under pressures of 3 to 5 MPa at temperatures of 180 to 250° C and cooling it under pressures of 7 to 10 MPa.

## Revendications

1. Masse de moulage stabilisée en polyéthylène présentant une masse molaire moyenne de 10⁵ à 10⁷ g/mole contenant un sel de zinc de dialkyldithiocarbamate comprenant des chaînes en C₆ à C₁₆ dans le groupement alkyle comme antioxydant et de l'α-tocophérol ou son dérivé comme stabilisateur.

2. Masse de moulage stabilisée selon la revendication 1, **caractérisée en ce que** la masse de moulage contient 0,01 à 1% en poids d'un sel de zinc de dialkyldithiocarbamate comprenant des chaînes en C₆ à C₁₆ dans le groupement alkyle comme antioxydant et 0,01 à 0,5% en poids d'α-tocophérol ou de son dérivé comme stabilisateur.

3. Masse de moulage stabilisée selon la revendication 1 ou 2, **caractérisée en ce que** la masse de moulage contient 3 à 40% en poids de fibres et/ou de poudre de graphite, de verre, de quartz ou de métaux comme charge et, le cas échéant, d'autres additifs usuels, tels que des pigments colorés, des lubrifiants, des stabilisateurs de transformation ou des agents ignifuges.

4. Procédé pour la préparation des masses de moulage selon l'une quelconque des revendications 1 à 3, **caractérisé en ce qu'**on mélange toutes les substances contenues ensemble et qu'on fritte le mélange, à des pressions d'environ 3 à 5 MPa à des températures d'environ 180 à 250°C, et qu'on refroidit le produit fritté sous des pressions d'environ 7 à 10 MPa.

5. Procédé pour la préparation des masses de moulage selon l'une quelconque des revendications 1 à 3, **caractérisé en ce qu'**on mélange d'abord, pour un lot maître de 0,1 à 10% en poids d'antioxydant, 0,1 à 5% en poids de stabilisateur et de reste polyéthylène, les composants individuels, qu'on règle avec du polyéthylène supplémentaire la concentration souhaitée, qu'on mélange le cas échéant avec d'autres agrégats, qu'on fritte ce mélange à des pressions de 3 à 5 MPa à des températures de 180 à 250°C et qu'on refroidit à des pressions de 7 à 10 MPa.
